Europäisches Patentamt

European Patent Office

Office européen des brevets

(11) Publication number: **0 171 173**
**B1**

(12) EUROPEAN PATENT SPECIFICATION

(45) Date of publication of patent specification: **21.09.88**

(21) Application number: **85304747.0**

(22) Date of filing: **03.07.85**

(51) Int. Cl.⁴: **A 61 C 8/00,** A 61 C 5/02,
A 61 K 6/00

(54) An article for the treatment of periodontal disease.

(30) Priority: **06.07.84 US 628308**

(43) Date of publication of application:
**12.02.86 Bulletin 86/07**

(45) Publication of the grant of the patent:
**21.09.88 Bulletin 88/38**

(84) Designated Contracting States:
**AT BE CH DE FR GB IT LI LU NL SE**

(56) References cited:
**FR-A-1 499 912**
**FR-A-2 306 672**

(73) Proprietor: **W.L. GORE & ASSOCIATES, INC.**
**555 Paper Mill Road P.O. Box 9329**
**Newark Delaware 19711 (US)**

(72) Inventor: **Scantlebury, Todd Van Ness**
**315 Woodland Drive**
**Flagstaff Arizona 86001 (US)**
Inventor: **Ambruster, Jeanne Bok**
**631 Toho Trail**
**Flagstaff Arizona 86001 (US)**
Inventor: **Campbell, Stephen Edward, D.D.S.**
**6530 E. Highland Road**
**Cave Creek Arizona 85331 (US)**

(74) Representative: **Taylor, Derek George et al**
**Mathisen, Macara & Co. The Coach House 6-8**
**Swakeleys Road**
**Ickenham Uxbridge UB10 8BZ (GB)**

Courier Press, Leamington Spa, England.

## Description

The present invention relates to an article for the treatment of periodontal disease, in particular an article for inhibiting periodontal pocket formation and healing periodontal defects.

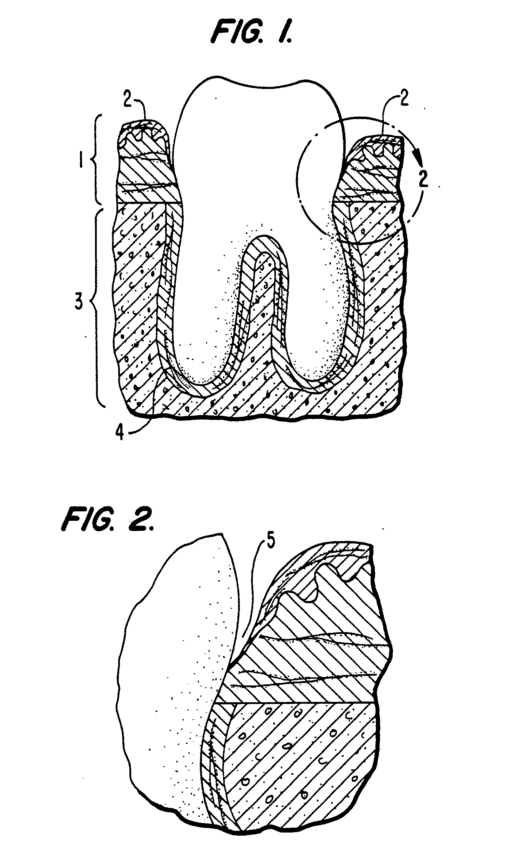

Periodontal disease is a disease of the periodontium, the tissues that invest and support the teeth. As shown in Figure 1 of the drawings these tissues include: gingiva 1, the soft gum tissue of the mouth; gingival epithelium 2, the protective surface layer of the gingiva which seals against the tooth where the tooth passes into the oral cavity; the cementum (not shown), a natural adhesive covering the tooth root; alveolar bone 3, the bone of the jaw surrounding the tooth root; and the periodontal ligament 4, connective tissue which suspends and supports the tooth between alveolar bone and the tooth root.

The gingival tissue surrounding a healthy adult tooth forms a sulcus 5 where it attaches to the tooth. In the early stages of periodontal disease the bacteria break down the attachment of the gingival epithelium to the tooth, forcing the epithelium to reattach apically (toward the root) away from infected tissue. Because the tissue is compromised by disease, the new attachment is weak. Further infection progressively moves the attachment apically until the tooth is surrounded by a loose sleeve of diseased gingiva creating a pocket which is much deeper than the normal sulcus. The loose sleeve, called a periodontal pocket 6, is difficult to clean because a tooth brush and floss cannot reach the bacteria and plaque which accumulate within the pocket. As disease extends the periodontal pocket, the cementum, periodontal ligament, and supporting alveolar bone are destroyed, leaving a periodontal defect filled with plaque and bacteria. Eventually the loss of the supporting periodontium leads to loss of the tooth.

Periodontal disease is the most common disease known to man, reportedly affecting 75 percent of the adult population and is the major reason for tooth loss after the age of 35. Fifty-five million teeth are lost to the disease each year in the United States.

Conventional treatment of periodontal defects consists of attempts to surgically alter the periodontal pocket morphology or obtain coronal (toward the crown) reattachment of the gingiva to the tooth.

Previous attempts to correct periodontal defects with synthetic materials have not provided for the attachment of gingival tissue to an implant on the tooth while controlling the apical migration of epithelium. For this reason, these techniques have met with only limited success. The present invention seeks to provide means by which periodontal disease can be treated by halting the apical migration of epithelium near the level maintained before the disease. The defect apical to the gingival attachment can then be healed in by the appropriate periodontal tissues.

It is known from US-A-4,007,494 to utilise a porous biocompatible material as a bone cap to cover the exposed end of an excised bone, and permit ingrowth of bone and other tissue, the cap including a biocompatible non-porous material. However, the specification does not disclose any article suitable for use in or teaching directed toward, the treatment of periodontal disease.

According to the present invention there is provided an article for the treatment of periodontal disease by promoting gingival tissue attachment to the article about a desired sulcus line comprising a member having first and second juxtaposed parts meeting at a boundary wherein said boundary is capable of at least partially encircling the perimeter of a tooth to be treated, said first part comprising a porous, biocompatible material capable of supporting ingrowth of gingival connective tissue and preventing the apical migration of gingival epithelium and configured so as to surround at least a portion of the perimeter of the tooth just apical to the desired sulcus line, with the porous material abutting the gingival connective tissue, said second part comprising a material impermeable to oral tissues and configured so as to surround at least a portion of the perimeter of said tooth apical to the first part so that after healing said first part is exposed to gingival epithelial tissue in the area of the desired sulcus line, and the second part abuts the gingival connective tissue.

As a result of the porous material being located between the gingival tissue and the tooth, the porous material fills with gingival connective tissue thus halting the apical downgrowth of gingival epithelium. As such, the area which previously comprised the periodontal pocket fills in with healthy periodontal tissue.

The material with which the present invention is concerned can be utilised in simpler forms of implant according to the invention.

According to one form the present invention provides an article for the treatment of periodontal disease comprising a length of porous material capable of supporting ingrowth of gingival connective tissue and preventing the apical migration of gingival epithelium, said length having a biocompatible, non-porous backing on a side capable of being wrapped around at least part of the surface of a tooth.

According to another form, the present invention provides an article for the treatment of periodontal disease comprising a length of porous material capable of being wrapped around at least part of the surface of a tooth, of supporting ingrowth of gingival connective tissues and of preventing apical migration of gingival epithelium, said length having at least one filament attached to each of its opposite ends for securing said material to the tooth. These filaments can serve to tie the porous material around a tooth.

The present invention can be used in a method for the treatment of periodontal disease comprising:

(a) temporarily separating the gingival tissue from a tooth surface in an area where periodontal disease is present;

(b) fixing an article in a laminar relationship to a portion perimeter of a tooth surface, wherein the article comprises a member having first and second juxtaposed parts meeting at a boundary wherein the boundary is capable of at least partially encircling the perimeter of the tooth to be treated, said first part comprising a porous, biocompatible material capable of supporting the ingrowth of gingival connective tissue and capable of preventing the apical migration of gingival epithelium, said first part further configured so as to surround a portion of the perimeter of the tooth just apical to the desired sulcus line with the porous part abutting the gingival connective tissue, and said second part being impermeable to oral tissues and configured so as to surround at least a portion of the perimeter of the tooth apical to the first surface, said second part positioned so as to leave, after healing, at least a portion of the surface of the first part exposed to gingival epithelial tissue in the area of the desired sulcus line and said second part abutting the gingival connection tissue, and

(c) repositioning the gingival tissue around the tooth and in contact with the article, with at least a segment of the first part of the article positioned to lie within the gingival tissue to be reattached to the tooth.

The accompanying drawings illustrate, by way of example, five embodiments of the invention. In the drawings:

Figure 1 is a mesial-distal (parallel with jawbone ridge) cross-sectional view of a healthy adult tooth and periodontium;

Figure 2 is a close-up view of the gingival sulcus of Figure 1;

Figure 3 is a mesial-distal cross-sectional view of a periodontally diseased tooth with periodontal pocket and defect;

Figure 4 is a mesial-distal cross-sectional view of the diseased tooth of Figure 3 repaired by use of the present invention;

Figure 5 is a three-dimensional view of uniaxially expanded polytetrafluoroethylene;

Figure 6 is a three-dimensional view of a first embodiment of an article according to the invention;

Figure 7 is a three-dimensional view of a second embodiment of the porous part of an article according to the invention;

Figure 8 is a three-dimensional view of a third embodiment of an article according to the invention;

Figure 9 is a three-dimensional view of a fourth embodiment of the article of the invention showing a first part of the article which is porous and a second part on of the article which is impermeable to oral tissues;

Figure 9A is a cross-sectional view of the fourth embodiment of Figure 9 taken along line A-B;

Figure 10 is a three-dimensional view of a fifth embodiment of the article of the invention showing a first part of the article which is porous and a second part of the article which is impermeable to oral tissues, and

Figure 10A is a cross-sectional view of the fifth embodiment of Figure 10 taken along line C-D.

Preferably, a periodontally diseased tooth is prepared for repair by exposing all diseased portions of the tooth and periodontium. For example, one or more buccal (toward cheek) or lingual (toward the tongue) flaps of gingiva 1 can be reflected away from the tooth root and surrounding bone. The exposed defect can then be cleaned by conventional periodontic techniques such as scaling or curetting. Topical antibiotics may be applied to the diseased sites to discourage the population of bacteria.

A strip of porous material (to be described in detail below) is placed against the tooth surface and should extend coronally to the level at which repair of the defect is desired to occur. The porous material is placed to the level on the tooth just apical of where it is desired for the gingival epithelium (sulcus) to end.

The porous material may be wrapped around the entire perimeter of the tooth. It is preferably placed around the portion of the tooth's perimeter where periodontal tissue attachment has been lost to disease. The porous material may be placed flat against the tooth surface. Preferably the porous material extends out from the tooth surface to cover any boney defects surrounding the tooth.

The porous material should be closely apposed to the tooth surface so that gingival epithelium cannot pass between the porous material and the tooth surface. The porous material may simply be placed between the gingival tissue and the tooth surface or it may be secured to the tooth surface.

The porous material may be secured to the tooth surface by adhering the material to the tooth with suitable dental adhesives. A suitable dental adhesive is non-toxic to surrounding tissues, is capable of forming an adhesive bond in an open oral environment, will remain for a sufficient amount of time to allow the defect to be repaired, and is capable of forming a bond between a porous surface and a tooth surface. Dental adhesives include, but are not limited to, zinc euginol, zinc phosphate, zinc silicophosphate, acrylic, glass ionomer, silicate and polycarboxylate cements.

The porous material may also be secured in place around the tooth through the use of sutures or filaments. Sutures may be used to lash the porous material to surrounding tissue and, thereby, hold it snugly against the tooth. Suture material may also be used to join the ends of porous material together to secure the porous material around the tooth. A filamentous material may be attached to the ends of the porous material and used to tie the porous material around the tooth. Such suture materials or filamentous materials must be biocompatible, i.e. must be materials which do not adversely affect oral tissue. Additionally, the sutures or filamentous materials should not act as a conduit for bacterial invasion nor should they resorb until sufficient time has elapsed to allow periodontal tissues to fill in the defect.

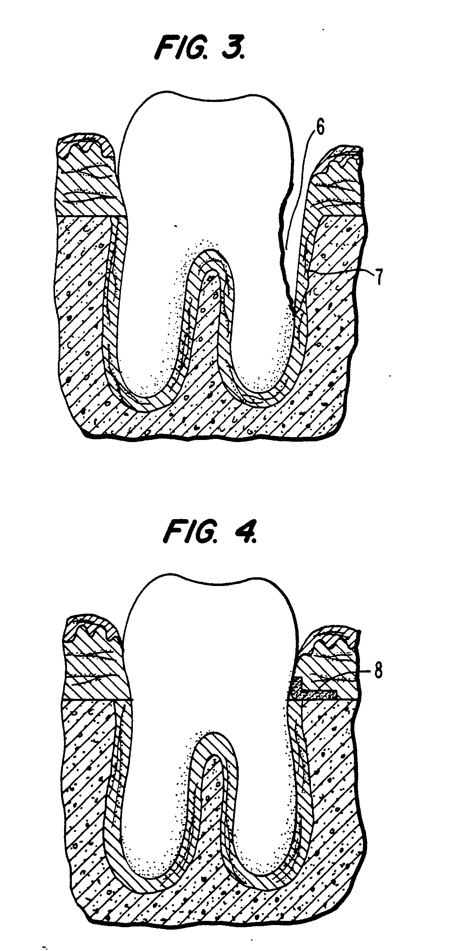

Gingival tissue is positioned against the porous material so that the porous material separates some portion of the gingiva from the tooth surface. The coronal portion of the porous material may extend coronal to the gingival tissue. Preferably, as depicted in Figure 4, the porous material 8 is completely covered with gingival tissue, leaving no portion of the porous material exposed to the oral cavity.

The porous material may remain in the mouth for the life of the patient. Alternatively, the porous material may be removed after a time sufficient for the periodontal tissues to fill the defect and attach to the tooth surface.

The porous material should be made from biocompatible materials. Preferably the porous material is soft and flexible so that it will conform to the curvature of the tooth and surrounding bone and not cause tissue necrosis in the tissues against which it is placed. Suitable biocompatible materials which can be made porous include, but are not limited to, silicones, polyurethanes, polyethylenes, polysulfones, polyacrylics, polycarboxylates, polyesters, polypropylenes poly(hydroxyethylmethacrylates), and perfluorinated polymers, such as fluorinated ethylene propylene, and polytetrafluoroethylene.

The above-mentioned may be made porous by any techniques known to those of ordinary skill in the art which will render the materials capable of supporting gingival connective tissue ingrowth while preventing apical migration of gingival epithelium. Such techniques include but are not limited to, sintering carefully controlled sizes of beads; combining the materials with a partially resorbable implant that would resorb or could be resorbed, *in vivo* or *in vitro*, to leave a porous surface; weaving or knitting fibers together to form a fabric-like material; or using a foaming agent during processing to cause bubbles to form and leave pores as the material hardens.

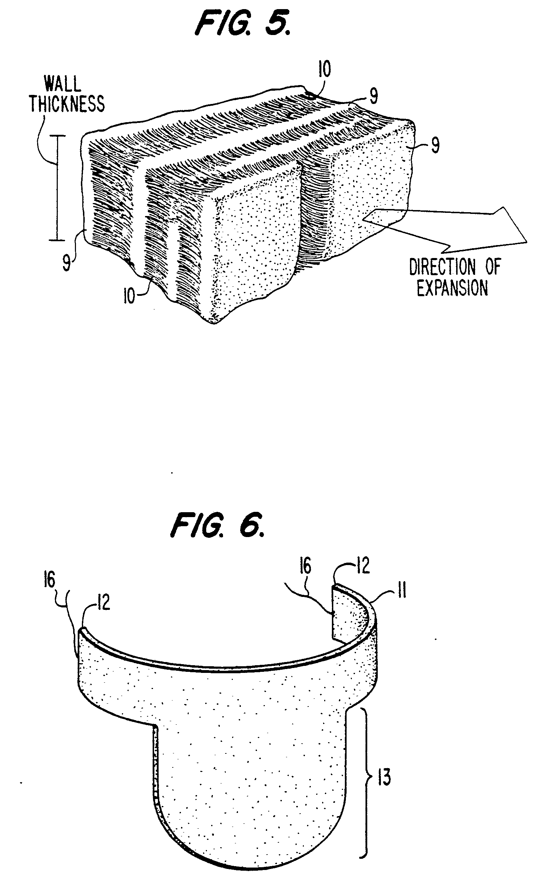

The porous material of the preferred embodiment is expanded polytetrafluoroethylene (expanded PTFE). Expanded PTFE is an extremely inert and biocompatible material with a history of medical implant use. U.S. Patent Numbers 3,953,566 and 4,187,390, teach methods for producing expanded PTFE and characterize its porous structure. The porous structure of expanded PTFE is further illustrated in Figure 5. The microstructure of expanded PTFE is a three-dimensional matrix of nodes 9, connected by fibrils 10.

The pore size of expanded PTFE can be characterised by determining the bubble point and the mean flow pressure of the material. Bubble point and mean flow pressure are measured according to the American Society for Testing and Materials Standard F316-80 using ethanol.

The density of expanded PTFE determines the amount of void space in the material which may become ingrown with connective tissue. The density of expanded PTFE is the ratio of the mass of a given sample of expanded PTFE to its volume.

The fibril length of expanded PTFE is defined herein as the average of ten measurements between nodes connected by fibrils in the direction of expansion. Although Figure 5 illustrates material expanded in one direction only, PTFE expanded in more than one direction is thought to be equally applicable to the invention. In order to measure the average fibril length of expanded PTFE, two parallel lines are drawn across a photomicrograph of about 40 to 50 times magnification of the surface of the material so as to divide the photograph into three equal areas. If the material has been uniaxially expanded, these lines are drawn in the direction of expansion (i.e. direction of orientation of fibrils). Measuring from left to right, five measurements of fibril length are made along the top line in the photograph beginning with the first node to intersect the line near the left edge of the photograph and continuing with consecutive nodes intersecting the line. Five more measurements are made along the other line from right to left beginning with the first node to intersect the line on the right hand side of the photograph. If the material is expanded in more than one direction, the lines are drawn and fibril lengths measured as above, except when a node is not attached by fibrils to a node intersecting the drawn line. In this case, the fibril length from the node to a node which creates the least angle with the drawn line is measured along the fibril' s axial orientation. The ten measurements obtained by this method are averaged to obtain the average fibril length of the material.

Materials with average fibril lengths greater than about 60 microns, preferably greater than 100 microns, ethanol bubble points or less than 2.0 psi (13.8 kPa), preferably less than 75 psi (5.2 kPa), ethanol mean flow pressure less than 10 psi (69 kPa), preferably less than 3.0 psi (21 kPa), and densities less than 1 g/cc and preferably 0.3 to 0.l g/cc enhance connective tissue ingrowth and are therefore preferred to use in the present invention.

When expanded PTFE is used as the porous material, it is preferred that a number of nodes 9 pass through the wall thickness of the expanded PTFE, as illustrated in Figure 5, to provide channels for tissue ingrowth and a wall resistant to crushing. Expanded PTFE without nodes passing through its wall thickeness is more easily crushed by forces of mastication, thereby decreasing the pore size, increasing the density, and compromising ingrowth. preferably, a majority of the nodes extend across the thickness dimension of the wall. In the prefered embodiments, an expanded PTFE gingival interface with a wall thickness of approximately 1mm is used.

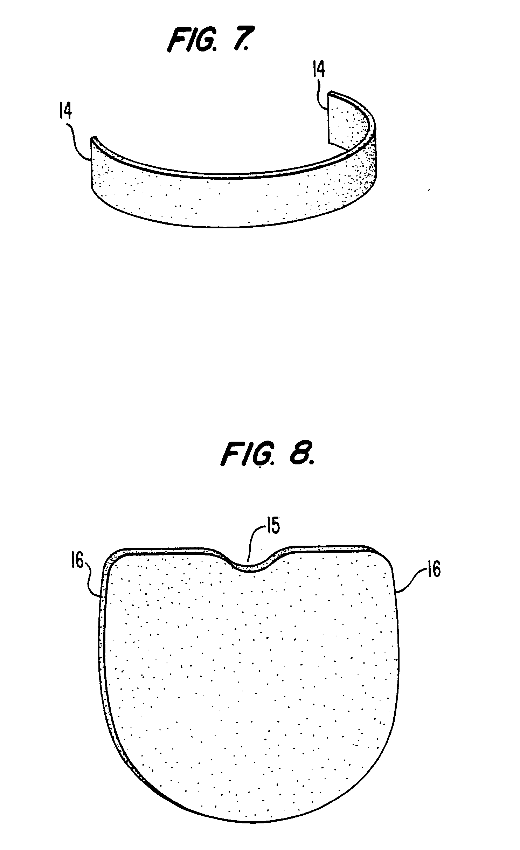

The expanded PTFE may be of several configurations. Figure 6 illustrates an "apron" configuration. The collar 11 of the apron is pulled snugly against the perimeter of the tooth, preferably by tying the ends 12 of the collar together with a pair of filaments 16 attached to ends 12 of the apron collar. The bib 13 of the apron is positioned coronally over boney defects adjacent to the tooth. Figure 7 illustrates a "collar" configuration. The collar of expanded PTFE is pulled snugly against the perimeter of the tooth, preferably tying the ends 14 of the collar together using filaments not shown. Figure 8 illustrates an additional preferred "patch" configuration. The notch 15 of the patch, shown in Figure 8, is placed snugly against the tooth, preferably by using suture material to lash the sides 16 of the patch to tissue surrounding the tooth.

The porous material may be treated or filled with biologically active substances such as antibiotics, fibrin, thrombin, and collagen. These substances may enhance connective tissue formation within the porous material and inhibit infection during healing.

According to one simple embodiment, the porous material is backed with a non-porous material (not shown in Figures 6-8) on the side of the porous material which juxtaposes the tooth surface. The non-porous material aids in retaining the open, porous structure of the porous material. The non-porous material also aids in sealing the porous material against the tooth surface by conforming to the irregularities of the surface. Suitable non-porous materials include, but are not limited to, the chemical substances listed above, and combinations thereof.

The non-porous material can also be used to secure the porous material to the tooth surface by serving as a surface which bonds to a tooth-adhering adhesive. In this embodiment, a suitable dental adhesive, as set forth above, may be applied to the tooth or the non-porous side of the porous material before adhering the material on to the tooth. In another embodiment, one component of a multi-component adhesive may be incorporated into the non-porous material. The adhesive or the remaining components of the adhesive are introduced immediately before the material is adhered to the tooth.

The porous material may be shaped as a collar, a patch or an apron, as set forth in the previous embodiments. Preferably, the porous material is shaped as an apron with the filaments serving as the "apron strings" as illustrated in Figure 6.

Preferably, the porous material is expanded PTFE as described above.

The filaments may be attached to the length of porous material by any method which provides an attachment capable of holding the porous material in place until sufficient ingrowth of periodontal tissue has occurred. Preferably, the filaments are tied or sewn to the porous material. If both the porous material and the filaments are comprised of expanded PTFE, the two may be joined by pressure and heat.

The present invention can also be used in an alternative preferred method for the treatment of periodontal disease. In this alternative method, the gingival tissue is separated from a tooth surface in an area where periodontal disease is present. An article comprising a member having first and second juxtaposed parts meeting at a boundary, wherein the boundary is capable of at least partially encircling the perimeter of a tooth to be treated, is fixed in a laminar relationship to a portion of the perimeter of the tooth surface. The first part comprises a porous, biocompatible material capable of supporting the ingrowth of gingival connective tissue and capable of preventing the apical migration of gingival epithelium. The first part is configured so as to surround at least a portion of the perimeter of the tooth about the desired sulcus line with the porous surface abutting the gingival epithelial tissue. The second part is impermeable to oral tissues and configured to surround at least a portion of the perimeter of the tooth apical to the desired sulcus. The second part is further positioned so as to leave at least a surface portion of the first part exposed to gingival epithelial tissue in the area of the desired sulcus line. The impermeable second part is intended to abut the gingival connective tissues.

Finally, the gingival tissues are repositioned around the tooth and in contact with the article, such that at least a segment of each of the first and second parts of the article are positioned between the gingival tissue and the tooth. preferably the gingival tissues are repositioned so that preferably the entire article is covered by gingival tissue.

In the articles intended for use in this method, the porous materials of the first part are contemplated to be similar to those discussed in detail in connection with the first method for treatment of periodontal disease discussed previously. In particular, it is preferred that the porous material be expanded PTFE.

Various materials are contemplated for use in the construction of the second part of the article. These materials include, but are not limited to, any of the porous biocompatible materials listed above made impermeable to oral tissues. It should be noted that the impermeable materials should be biocompatible in the sense that they do not cause inflammation of the oral tissues or react adversely with tissues to inhibit the healing process.

In one embodiment, various lamination methods might be employed to join the second part of the article to the first part. Biocompatible adhesives including, but not limited to, polyurethanes or silicones may be used to join the two parts.

In an alternative embodiment, porous materials suitable for the first part might be rendered impermeable to oral tissues in areas desired for the second part by either coating or filling the desired areas with, for example, silicones or urethanes or compressing the desired areas to make them less porous.

In a preferred embodiment, expanded PTFE capable of supporting gingival connective tissue ingrowth is utilized for the first and second parts. To construct the second part, the PTFE is rendered impermeable to tissue ingrowth in desired areas by the application of heat and pressure. In this embodiment, the porous expanded PTFE of the first part is an integral construction with the impermeable PTFE of the second part. The second part is preferably formed by the application of plates heated between 300 and 400°F (149° and 204°K) and pressed against the porous expanded PTFE in the desired areas. In this embodiment, because the second part is created by compressing the expanded PTFE of the first part, no means for attachment of the first and second parts of the member are required.

The articles for use in this alternative method are placed in the area of the periodontal defect as set forth above, with care being taken to ensure that the first, porous part of the article is placed coronal to the second, impermeable part. This article may be secured in the area of the defect, if so desired, with various biocompatible glues, sutures or filaments.

Alternatively, either or both of the first and second parts of the articles used in conjunction with this alternative method may be backed with any of the variety of non-porous substances previously disclosed. As noted above, such a backing will serve to retain the open structure of the porous material of the first part of the article and may assist in the securing of the articles into the area of the defect.

The advantage of this second method is that the article may be removed easily. After desired healing of periodontal tissue has taken place, the first part of the article, which is ingrown with connective tissue and has stopped the apical migration of epithelium, is separated from the gingiva. The second part of the article can then be removed easily, as it is impermeable to tissue ingrowth.

It is generally contemplated that the article for use in this method will be removed from the perimeter of the tooth at about four weeks after implantation, although the article may be removed at any time the desired periodontal structures have regenerated. The article may also be kept in place indefinitely. Upon removal of the article, it is believed that periodontal structures will have regenerated sufficiently to provide improved periodontal health for the tooth.

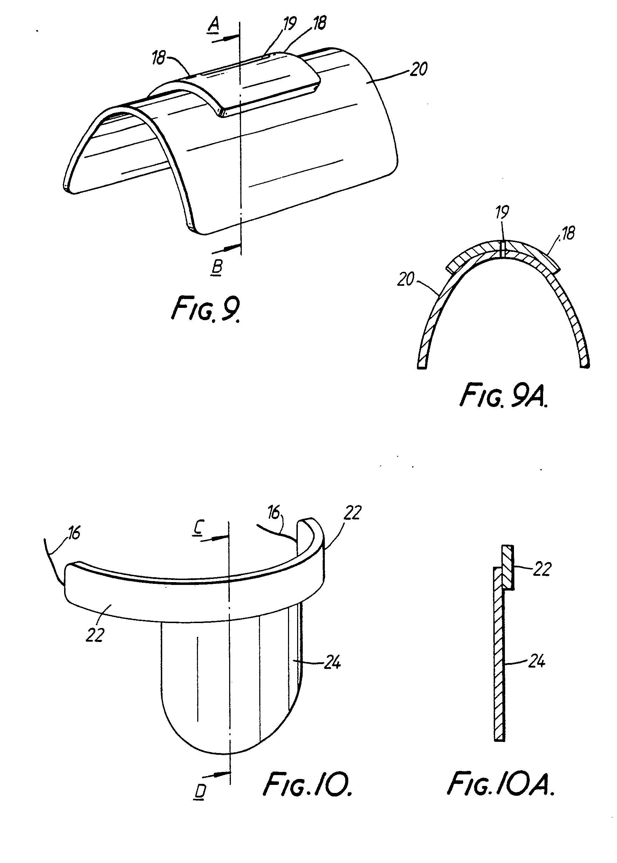

Two preferred embodiments of this article are shown in Figures 9 and 10. In Figure 9, the article has a "poncho" shape. In this embodiment, the center 18 of the poncho forms the first, porous part and the outer circumferencial portion 20 of the poncho forms the second, impermeable part. A slit 19 or hole may be rendered in the first part 18 of the article so that the article may be placed over the tooth with part 18 surrounding the perimeter of the tooth in the area of the desired sulcus line.

In the alternative embodiment of Figure 10, the article has an apron shape suitable for forming into a collar wherein the first, porous part 22 of the article and the second part 24 of the article are depicted. When the article as shown in Figure 10 is used in the above method, suture material 16 may be used to secure the article around the perimeter of the tooth.

The articles of the present invention are preferably sterilized prior to insertion into a periodontal pocket. Preferably, the articles are sterilized and are contained in a package, the interior environment of which is sterile.

The following examples are designed to elucidate the teachings of the present invention.

Example 1

Expanded PTFE aprons similar to those illustrated in Figure 6 were implanted in dogs.

The expanded PTFE for the aprons was made according to the description in U.S. Patent Numbers 4,187,390 and 3,953,566 as follows:

A mixture of PTFE resin in a liquid lubricant was extruded into a tubular form. The extruded tubular form was dried for approximately 96 hours at about 300°C, which removed the lubricant. The tubular form, held at a temperature of about 195°C, was then stretched at a constant velocity along the central axis of the tube at a rate of about 75% per second until it was approximately 10 times its original length, where rate was defined as

$$\frac{lf - li}{l(t)} \times 100\%$$

and lf = final length, li = initial length, and t = total time of expansion. Subsequent to stretching, the tube was restrained longitudinally and heat treated at about 375°C for about 75 seconds. The expanded PTFE tube was then slit longtitudinally through its wall and laid flat. The wall was about 1mm thick and had nodes passing through its wall thickness. The ethanol bubble point of the material was about 0.5 psi (3.4kPa) and the ethanol mean flow pressure was about 1.0 psi (6.9kpa). The average fibril length was about 200 microns, and the density was approximately 0.2 g/cc.

The bib and collar portion of the apron were cut from the expanded PTFE so that the collar was about 2cm long and 2mm wide, and the bib extended out from the collar about a centimeter. A 6.0 GORE-TEX (trade mark) Expanded PTFE Suture filament, obtained from W. L. Gore & Associates, Flagstaff, Arizona, was passed through each end of the apron collar. All assembly of the apron took place in a clean room environment. The apron was sterilized prior to implantation.

At implantation, buccal and lingual periosteal flaps were reflected around dog premolar teeth. About 2mm of crestal bone was removed circumferentially from the teeth to expose a root surface against which the aprons could be applied. Either a three wall mesial defect or a one wall buccal defect was created adjacent to the teeth using burrs and chisels. The collar of the apron was placed around the perimeter of the tooth with the bib positioned coronally over the created boney defect. The ends of the GORE-TEX (trade mark) Expanded PTFE sutures were then tied together on the side of the tooth opposite the defect drawing the ends of the collars together and tightening the apron around the tooth. Any excess suture was trimmed off, and the gingival flaps were repositioned over the apron by suturing them in place. A total of five implants were placed in two dogs.

Beginning one week after surgery the implant sites were assessed clinically. After initial healing, probing revealed in all cases a healthy appearing gingiva attached to the apron forming a 2 to 3mm deep, normal appearing sulcus. In one case, a small portion of the apron was inadvertently left exposed to the oral cavity, but all surrounding tissue appeared healthy and healed into the remainder of the apron.

Two implants, including the above mentioned case, were harvested at 20 and 30 days and assessed histologically. The expanded PTFE collar and bib, in all areas except that exposed to the oral cavity, appeared filled with connective tissue.

Epithelium apparently halted its apical migration at or directly coronal to the expanded PTFE, and new bone had filled the created defects.

### Example 2

Expanded PTFE collars as illustrated in Figure 7 backed with medical grade silicone and polyurethane were implanted in dogs. The medical grade silicone, obtained from Dow-Corning Corporation, and polyurethane, Cardiomat 610 (trade mark) obtained from Kontron Cardiovascular Inc., were applied to the expanded PTFE as a thin backing and allowed to cure. The expanded PTFE had an ethanol bubble point below about 0.75 psi (5.2kPa), an ethanol mean flow pressure below about 3 psi (21kPa), an average fibril length greater than about 100 microns, a density of about 0.3 to 0.1 g/cc, and a wall thickness of about 1mm.

The backed, expanded PTFE was cut into strips (collars) approximately 2mm wide and 2cm long. The collars were sterilized and implanted in premolar sites according to the procedure detailed in example 1 without creating defects. The silicone or polyurethane backed side of the collars was placed against the tooth, and each collar was secured around a tooth by tying its ends together with GORE-TEX (trade mark) Expanded PTFE Suture. Four implants were retrieved between 19 and 32 days after surgery for histological analysis.

Probing of the implants prior to retrieval revealed a normal appearing sulcus with no inflammation and gingiva attached to the collar. Microscopic examination of the implant revealed that the polyurethane and silicone backings were in most places directly apposed to the tooth root. The expanded PTFE had filled with connective tissue, and the gingival epithelium had attached to the connective tissue within the collar, apparently halting its apical migration.

### Example 3

Expanded PTFE with a urethane backing was adhered to dog teeth using a conventional glass ionomer dental cement. The expanded PTFE had an ethanol bubble point below 0.75 psi (5.2kPa), and ethanol mean flow pressure below 3 psi (21kpa), an average fibril length greater than 100 microns, a density of 0.3 to 0.1 g/cc, and a wall thickness of about 1mm. It was backed with a mixture of glass ionomer powder, Chembond (trade mark) Glass Ionomer Cement obtained from the L. D. Caulk Company, and liquid urethane, Cardiomat 610 (trade mark) polymer. About 1.2cc of the urethane was mixed with about 1 gram of the powder and spread in a thin coating on one side of the expanded PTFE. The backing was allowed to cure for about 24 hours. The backed, expanded PTFE was sterilized.

A small piece of the backed material approximately 1cm x 0.5cm was adhered to the right maxillary canine root in a dog. After a gingival flap had been reflected, the glass ionomer cement was mixed according to the manufacturer's instructions and placed on the root. The backed side of the expanded PTFE was pressed into the cement before the cement had cured, and any excess cement was removed. After the adhesive had cured, the gingival flap was repositioned over the expanded PTFE and sutured in place.

Approximately one month after surgery, the implant was retrieved. Microscopic examination revealed that the backing had not remained completely attached to the tooth but that the expanded PTFE had filled with healthy, vascularized connective tissue. Gingival epithelium appeared to be attached to the connective tissue, apparently halting epithelial apical migration.

### Example 4

An apron like that described in Example 1 and two collars like those described in Example 2 were backed with a thin line of either silicone or urethane elastomer. Approximately 0.5mm wide lines of Cardiomat 610 (trade mark) Urethane or Dow Corning medical grade silicone were forced from a syringe on to the expanded PTFE along the length of collar. The lines were placed on the side of the collar meant to fit against the tooth. The expanded PTFE collar was held slack to about 75% of its fully extended length during the application of the elastomer so that after the collar was stretched around the tooth and the ends of the collar tied together with sutures, the elastomer held the collar against the tooth as an elastic waistband holds trousers against the waist.

Each implant was sterilized and implanted in premolar sites in a dog with the bib of the apron covering a defect as described in Example 1 and the collars placed in sites with no defects. In all cases after an initial week of healing, probing revealed healthy appearing gingiva attached to the expanded PTFE forming a normal appearing sulcus adjacent to the tooth. Histological analysis showed the epithelium apparently halting its apical migration at or directly coronal to expanded PTFE filled with connective tissue. New bone had healed in the defect apical to the apron.

### Example 5

Expanded PTFE was cut into small patches approximately 12mm x 12mm as illustrated in Figure 8. The patches had an ethanol bubble point below about 0.75 psi (5.2kPa), an ethanol mean flow pressure below about 3 psi (21kPa), an average fibril length greater than 100 microns, a density of 0.3 to 0.1 g/cc, and a wall thickness of about 1mm. After gingival flaps had been reflected, three wall mesial defects were created adjacent to the roots of two first molars in one dog and the fourth premolar in the mandible of another dog. The patches were fitted over the boney defects and conformed to the boney ridge with the root of the treated tooth resting in the notch of the patch. The gingival flaps were repositioned over the patches and sutured in place. The molar implants were retrieved 19 and 20 days after surgery, and the premolar implant was retrieved 30 days following surgery.

In all sites approximately 2mm of expanded PTFE remained exposed to the oral cavity adjacent to the mesial aspect of the treated teeth. Histological analysis revealed that gingival tissue had filled a space between the notch of the patch and the tooth. New bone had filled the defects, and the patches had apparently inhibited the growth of gingival tissue into the defect space. The portions of expanded PTFE which had not become exposed to the oral environment were healed in with healthy, vascularized connective tissue. Epithelium had apparently halted its apical migration at the interface between the exposed and healed portions of expanded PTFE.

This treatment method was somewhat unsuccessful because gingival tissue managed to grow between the patch and the tooth surfaces. It may be possible to appose the notch of such a patch more tightly against the tooth by lashing the patch to adjacent tissue with sutures or by other suitable means. Such a patch might enable a normal sulcus to be formed around the tooth without the formation of gingival tissue between the patch and tooth or the exposure of the patch to the oral cavity. The boney defect would be filled in by the appropriate tissues of the periodontium as demonstrated in the example.

### Example 6

Expanded PTFE was adhered to a tooth using acrylic cement. The expanded PTFE had an ethanol bubble point below 0.75 psi (5.2kPa), and ethanol mean flow pressure below 3 psi (21kPa), an average fibril length greater than 100 microns, a density of 0.3 to 0.1 g/cc, and a wall thickness of about 1mm. Concise (trade mark) and Silar (trade mark) cements were used as supplied by Dental Products/3M.

After buccal gingival flaps had been reflected from the maxillary canine of a dog, the exposed root was notched approximately 0.5mm deep and 2mm wide directly coronal to the bone surrounding the root from the mesial to the distal aspect of the exposed root. Silar (trade mark) paste mixed according to the manufacturer's instructions was then used to fill the notch.

A strip of the expanded PTFE, which had been sterilized, was cut approximately 2mm wide and long enough to extend from the mesial to the distal aspect of the exposed root. Concise (trade mark) resin liquids were mixed with Concise (trade mark) paste components forming a paste which could be spread and adhered on one side of the expanded PTFE strip. The pasted side of the expanded PTFE strip was placed against the Silar (trade mark) filled notch on the root. The adhesive was allowed to set, and the gingival flap was repositioned over the expanded PTFE strip and sutured in place.

After 40 days the implant was retrieved. The expanded PTFE strip appeared well attached to the tooth. Epithelium appeared to end against the expanded PTFE, but gingival tissue surrounding and within the expanded PTFE exhibited an apparent adverse reaction to the adhesive.

An adhesive system which would bond an expanded PTFE strip to the tooth and which would be biocompatible with the oral tissue would be a more viable treatment modality.

### Example 7

Ponchos and aprons, as illustrated in Figures 9 and 10, were made by laminating two structures of expanded PTFE. GORE TEX (trade mark) expanded PTFE Surgical Membrane of 0.1 mm thickness obtained from W. L. Gore & Associates, Incorporated was cut into 40 x 25 mm rectangles to form the portion of the ponchos and aprons impermeable to tissue ingrowth. Expanded PTFE, capable of supporting the ingrowth of gingival connective tissue and preventing the migration of gingival epithelium, was cut into smaller rectangles, either 10 x 20 mm, 8 x 20 mm or 5 x 20 mm.

These smaller rectangles of expanded PTFE had an ethanol bubble point below about 0.75 psi (5.2kPa), an ethanol mean flow pressure below about 3 psi (20.7kPa), an average fibril length greater than about 100 microns, a density of about 0.3 to 0.1 g/cc, and a wall thickness of about 0.5 mm. The smaller rectangles were laminated to the Surgical Membrane in roughly the center of the Surgical Membrane rectangles using a thin layer of either biocompatible urethane or silicone adhesive placed between the two expanded PTFE structures. The biocompatible silicone adhesive was obtained from Dow Corning Corporation, and the biocompatible urethane adhesive was a segmented polyether polyurethane obtained from Ethicon Incorporated.

After the adhesive had cured, the ponchos and aprons were steam sterilized in preparation for implantation in dogs. During surgery, buccal and lingual periosteal flaps were reflected around dog premolar teeth. Up to 7 mm of crestal bone, ligament and cementum were removed to create horizontal defects with furcation involvement. The aprons or ponchos were implanted with the surgical membrane material trimmed to cover the prepared defects. The gingival flaps were repositioned over the implanted material by suturing the flaps in place.

Shaping of the pieces into either ponchos or aprons took place at the time of the surgery by using scalpel or sharp scissors. If a poncho was to be implanted, a slit was made through the laminate within the area of smaller rectangle of expanded PTFE. The slit was sized to fit over the crowns of the dogs' teeth and fit snugly around the tooth below the cemento-enamel junction (CEJ). The expanded PTFE capable of supporting the ingrowth of gingival connective tissue formed a collar around the tooth coronal to the Surgical Membrane portion which formed a skirt covering the defect. Aprons were formed by cutting a collar shape out of the smaller expanded PTFE rectangle and a bib shape out of the surgical membrane portion. The collar portions had extended tabs which were wrapped around the circumference of the tooth and drawn together against the tooth by means of absorbable suture. The collars (capable of supporting gingival connective tissue ingrowth) were placed coronal to the bib portion of the aprons which covered the defects.

Approximately 20 to 30 days after surgery, a small incision around the teeth or, in some cases, a split thickness flap was used to separate tissue which had ingrown into the portion of the implants capable of supporting gingival connective tissue ingrowth. The entire implants, including collars and Surgical Membrane, were removed, and gingival tissue was reapposed to the tooth with suture where necessary.

Of eight teeth implanted, two were removed at 37 days, one was removed at 49 days, and one was removed at 61 days post-surgery for microscopic examination. The remaining implants remained *in vivo* for clinical evaluation.

All implant sites showed no sign of inflammation and displayed normal sulcus depths. There were no gingival pockets or other signs of apical epithelial migration. The teeth were all immobile. In one case, a tooth that had been made mobile by defect creation at the time of surgery became immobile after healing evidence that periodontium had regenerated to support the tooth.

## Claims

1. An article for the treatment of periodontal disease by promoting gingival tissue attachment to the article about a desired sulcus line comprising a member having first and second juxtaposed parts meeting at a boundary wherein said boundary is capable of at least partially encircling the perimeter of a tooth to be treated, said first part comprising a porous, biocompatible material capable of supporting ingrowth of gingival connective tissue and preventing the apical migration of gingival epithelium and configured so as to surround at least a portion of the perimeter of the tooth just apical to the desired sulcus line, with the porous material abutting the gingival connective tissue, said second part comprising a material impermeable to oral tissues and configured so as to surround at least a portion of the perimeter of said tooth apical to the first part so that after healing said first part is exposed to gingival epithelial tissue in the area of the desired sulcus line, and the second part abuts the gingival connective tissue.

2. An article according to claim 1 wherein said first and second parts are integrally formed.

3. An article according to claim 2 wherein said first and second parts are a laminar construction.

4. An article according to claim 2 wherein said first part of said article is a biocompatible, porous material selected from the group consisting of silicones, polyurethanes, polyethylenes, polysulfones, polyacrylics, polycarboxylates, polyesters, polypropylenes, poly(hydroxyethylmethacrylates) and perfluorinated polymers.

5. An article according to any preceding claim wherein said porous material is expanded polytetrafluoroethylene.

6. An article according to claim 5 wherein said polytetrafluoroethylene of said first part has an average fibril length of greater than 60 microns, an ethanol bubble point of less than 2.0 psi (13.8kPa), an ethanol mean flow pressure of less than 10 psi (69kPa) and a density of less than 1 g/cc.

7. An article according to claim 1 wherein said polytetrafluoroethylene has an average fibril length of greater than 100 microns, an ethanol bubble point of less than 0.75 psi (5.2kPa), an ethanol mean flow pressure of less than 3.0 psi (20.7kpa) and a density of 0.3 to 0.1 g/cc.

8. An article according to claim 1 wherein said second part is compressed expanded polytetrafluoroethylene.

9. An article according to claim 1 having a non-porous backing.

10. An article for the treatment of periodontal disease comprising a length of porous material capable of supporting ingrowth of gingival connective tissue and preventing the apical migration of gingival epithelium, said length having a biocompatible, non-porous backing on a side capable of being wrapped around at least part of the surface of a tooth.

11. An article according to claim 9 or claim 10 wherein said non-porous backing is polyurethane.

12. An article according to claim 9 or claim 10 wherein said non-porous backing is silicone.

13. An article for the treatment of periodontal disease comprising a length of porous material capable of being wrapped around at least part of the surface of a tooth, of supporting ingrowth of gingival connective tissues and of preventing apical migration of gingival epithelium, said length having at least one filament attached to each of its opposite ends for securing said material to the tooth.

14. An article according to one of claims 1 to 9 which is in the shape of an poncho, the center of which is formed by the porous material and the outer circumference of which is formed by the impermeable material.

15. An article according to any preceding claim which is in the shape of an apron.

16. An article according to any preceding claim which incorporates a biologically active substance.

17. An article according to any one of claims 1 to 9 wherein said impermeable part has thereon an adhesive component.

18. An article according to claim 17 wherein said adhesive component comprises a glass ionomer powder and urethane.

19. An article of manufacture for the treatment of periodontal disease by supporting preferential tissue ingrowth, comprising a first part consisting of a length of sintered porous polytetrafluoroethylene material capable of supporting ingrowth of gingival connective tissue and preventing the apical migration of gingival epithelium, and a second part adjoining the first part, this second part consisting of sintered porous polytetrafluoroethylene material compressed relative to that of the first part, for preventing tissue ingrowth.

**Patentansprüche**

1. Artikel zur Behandlung von parodontaler Erkrankung durch Fördern der Befestigung des Zahnfleischgewebes am Artikel über eine erwünschte Sulcuslinie umfassend ein Element mit einem ersten und einem zweiten nebeneinanderliegenden Teil, die sich an einer Grenzlinie treffen, welche Grenzlinie zumindest teilweise den Umfang eines zu benandelnden Zahnes umschließen kann, wobei der erste Teil aus einem porösen bioverträglichen Material besteht, das imstande ist, das Einwachsen von Zahnfleischbindegewebe zu unterstützen und die apikale Wanderung von Zahnfleischepithel zu verhindern, und so ausgebildet ist, um zumindest einen Teil des Umfaungs des Zahnes unmittelbar apikal der gewünschten Sulcuslinie zu umgeben, wobei das poröse Material an das Zahnfleischbindegewebe angrenzt, und der zweite Teil aus einem Material besteht, das für Mundgewebe undurchlässig ist und so ausgebildet ist, um zumindest einen Teil des Umfangs des genannten Zahns apikal dem ersten Teil zu umgeben, so daß nach dem Heilen der erste Teil dem Zahnfleischepithelgewebe im Bereich der gewünschten Sulcuslinie ausgesetzt ist und der zweite Teil an das Zahnfleischbindegewebe angrenzt.

2. Artikel nach Anspruch 1, worin der erste und der zweite Teil intergrierend ausgebildet sind.

3. Artikel nach Anspruch 2, worin der erste und der zweite Teil eine laminare Konstruktion sind.

4. Artikel nach Anspruch 2, dadurch gekennzeichnet, daß der erste Teil des Artikels ein bioverträgliches, poröses Material ausgewählt aus der Gruppe bestehend aus Silikonen, Polyurethanen, Polyäthylenen, Polysulfonen, Polyacrylen, Polycarboxylaten, Polyestern, Polypropylenen, Poly(hydroxyäthylmethacrylaten) und perfluorierten Polymeren ist.

5. Artikel nach einem vorhergehenden Anspruch, worin das poröse Material expandiertes Polytetrafluoräthylen ist.

6. Artikel nach Anspruch 5, worin das Polytetrafluoräthylen des ersten Teiles eine mittlere Fibrillenlänge von größer als 60 μum, einen Äthanolblasenpunkt von weniger als 2,0 psi (13,8 kPa), einen mittleren Äthanolströmungsdruck von weniger als 10 psi (69 kPa) und eine Dichte von weniger als 1 g/cm³ aufweist.

7. Artikel nach Anspruch 1, worin das Polytetrafluoräthylen eine mittlere Fibrillenlänge von größer als 100 μum, einen Äthanolblasenpunkt von weniger als 0,75 psi (5,2 kPa), einen mittleren Äthanolströmungsdruck von weniger als 3,0 psi (20,7 kPa) und eine Dichte von 0,3 bis 0,1 g/cm³ aufweist.

8. Artikel nach Anspruch 1, worin der zweite Teil komprimiertes expandiertes Polytetrafluoräthylen ist.

9. Artikel nach Anspruch 1, der eine nicht-poröse Unterlage aufweist.

10. Artikel zur Behandlung von parodontaler Erkrankung umfassend eine Länge aus porösem Material, das das Einwachsen von Zahnfleischbindegewebe unterstützen und die apikale Wanderung von Zahnfleischepithel verhindern kann, welche Länge eine bioverträgliche, nicht-poröse Unterlage auf einer Seite aufweist, die zumindest um einen Teil der Oberfläche eines Zahns gewickelt werden kann.

11. Artikel nach Anspruch 9 oder 10, worin die nicht-poröse Unterlage Polyurethan ist.

12. Artikel nach Anspruch 9 oder 10, worin die nicht-porose Unterlage Silikon ist.

13. Artikel zur Behandlung von parodontaler Erkrankung umfassend eine Länge aus porösem Material, das zumindest um einen Teil der Oberfläche eines Zahnes gewickelt werden, das Einwachsen von Zahnfleischbindegewebe unterstützen und die apikale Wanderung von Zahnfleischepithel verhindern kann, welche Länge mindestens einen Faden aufweist, der zum Schützen des Materials am Zahn an jedem der gegenüberliegenden Enden derselben befestigt ist.

14. Artikel nach einem der Ansprüche 1 bis 9, der in Form eines Poncho ist, dessen Zentrum aus dem porösen Material und dessen Außenumfang aus dem undurchlässigen Material gebildet ist.

15. Artikel nach einem vorhergehenden Anspruch, der in Form einer Schürze ist.

16. Artikel nach einem vorhergehenden Anspruch, der eine biologisch aktive Substanz aufweist.

17. Artikel nach einem der Ansprüche 1 bis 9, worin sich auf dem undurchlässigen Teil eine Klebstoffkomponente befindet.

18. Artikel nach Anspruch 17, worin die Klebstoffkomponente ein Glasionomerpulver und Urethan umfaßt.

19. Fertigungsartikel zur Behandlung von parodontaler Erkrankung durch Unterstützen des bevorzugten Gewebeeinwuhses, umfassend einen ersten Teil bestehend aus einer Länge von gesintertem porösen Polytetrafluoräthylenmaterial, das imstande ist, das Einwachsen von Zahnfleischbindegewebe zu unterstützen und die apikale Wanderung von Zahnfleischepithel zu verhindern, und einen zweiten Teil, der an den ersten Teil angrenzt, wobei dieser zweite Teil aus gesintertem porösen Polytetrafluoräthylenmaterial besteht, das in bezug auf jenes des ersten Teils komprimiert ist, um Einwachsen von Gewebe zu verhindern.

## Revendications

1. Article pour le traitement des affections périodontales permettant de promouvoir la fixation de tissu gingival à l'article autour d'une ligne en sillon désirée, comprenant un élément comportant des première et seconde parties juxtaposées se rencontrant en une jonction, cette jonction pouvant au moins partiellement encercler le périmètre d'une dent à traiter, cette première partie comprenant une matière biocompatible, poreuse pouvant supporter la croissance intérieure de tissu connectif gingival et empêcher la migration apicale d'épithélium gingival et étant configurée de manière à entourer au moins une partie du périmètre de la dent juste apicale à la ligne en sillon désirée, la matière poreuse venant s'appuyer contre le tissu connectif gingival, cette seconde partie comprenant une matière imperméable aux tissus oraux et étant configurée de manière à entourer au moins une partie du périmètre de la dent précitée apicale à la première partie de telle sorte qu'après la cicatrisation la première partie soit exposée à du tissu épithélial gingival dans la zone de la ligne en sillon désirée, la seconde partie venant s'appuyer contre le tissu connectif gingival.

2. Article suivant la revendication 1, caractérisé en ce que les première et seconde parties sont formées solidairement.

3. Article suivant la revendication 2, caractérisé en ce que les première et seconde parties constituent un agencement laminaire.

4. Article suivant la revendication 2, caractérisé en ce que la première partie de l'article est une matière poreuse, biocompatible choisie dans le groupe comprenant les silicones, les polyuréthannes, les polyéthylènes, les polysulfones, les polyacryliques, les polycarboxylates, les polyesters, les polypropylènes, les poly(hydroxyéthyméthacrylates) et les polymères perfluorés.

5. Article suivant l'une quelconque des revendications précédentes, caractérisé en ce que la matière poreuse est du polytétrafluoréthylène expansé.

6. Article suivant la revendication 5, caractérisé en ce que le polytétrafluoréthylène de la première partie a une longueur de fibrille moyenne supérieure à 60 microns, un point de bulle dans l'étbanol inférieur à 2,0 livres/pouce carré (13,8 kPa), une pression d'écoulement moyenne dans l'éthanol inférieure à 10 livres/pouce carré (69 kPa) et une densité inférieure à 1 g/cc.

7. Article suivant la revendication 1, caractérisé en ce que le polytétrafluoréthylène a une longueur de fibrille moyenne supérieure à 100 microns, un point de bulle dans l'éthanol inférieur à 0,75 livre/pouce carré (5,2 kpa), une pression d'écoulement moyenne dans l'éthanol inférieure à 3,0 livres/pouce carré (20,7 kPa) et une densité de 0,3 à 0,1 g/cc.

8. Article suivant la revendication 1, caractérisé en ce que la seconde partie est du polytétrafluoréthylène expansé comprimé.

9. Article suivant la revendication 1, caractérisé en ce qu'il comporte un renfort non poreux.

10. Article pour le traitement des affections périodontales, comprenant une longueur de matière poreuse pouvant supporter la croissance intérieure de tissu connectif gingival et empêcher la migration apicale d'épithélium gingival, cette longueur comportant un renfort non poreux, biocompatible sur une face pouvant être enroulé autour d'au moins une partie de la surface d'une dent.

11. Article suivant l'une ou l'autre des revendications 9 et 10, caractérisé en ce que le renfort non poreux est du polyuréthanne.

12. Article suivant l'une ou l'autre des revendications 9 et 10, caractérisé en ce que le renfort non poreux est de la silicone.

13. Article pour le traitement des affections périodontales, comprenant une longueur de matière poreuse pouvant être enroulée autour d'au moins une partie de la surface d'une dent, supportant la croissance intérieure de tissus connectifs gingivaux et empêchant la migration apicale d'épithélium gingival, cette longueur comportant au moins un filament attaché à chacune de ses extrémités pour fixer la matière à la dent.

14. Article suivant l'une quelconque des revendications 1 à 9, caractérisé en ce qu'il a la forme d'un poncho, dont le centre est formé par la matière poreuse et dont la circonférence extérieure est formée par la matière imperméable.

15. Article suivant l'une quelconque des revendications précédentes, caractérisé en ce qu'il a la forme d'un tablier.

16. Article suivant l'une quelconque des revendications précédentes, caractérisé en ce qu'il comprend une substance biologiquement active.

17. Article suivant l'une quelconque des revendications 1 à 9, caractérisé en ce que la partie imperméable comporte un composant adhésif.

18. Article suivant la revendication 17, caractérisé en ce que le composant adhésif comprend une poudre d'ionomère vitreux et de l'uréthanne.

19. Article de fabrication pour le traitement des affections périodontales destiné à supporter une croissance intérieure de tissu préférentiel, comprenant une première partie constituée par une longueur de matière de polytétrafluoréthylène poreuse, frittée pouvant supporter la croissance intérieure de tissu connectif gingival et empêcher la migration apicale d'épithélium gingival, et une seconde partie contiguë à la première partie, cette seconde partie étant constituée par une matière de polytétrafluoréthylène poreuse, frittée comprimée par rapport à la matière de la première partie, pour empêcher la croissance intérieure de tissu.

## FIG. I.

## FIG. 2.

## FIG. 3.

## FIG. 4.

## FIG. 5.

WALL
THICKNESS

DIRECTION OF
EXPANSION

## FIG. 6.

## FIG. 7.

14

14

## FIG. 8.

15

16

16

*A* 19 18

18 20

*B*

*FIG. 9.*

19 18

20

*FIG. 9A.*

16 *C* 16 22

16

22

24

*D* *FIG. 10.*

22

24

*FIG. 10A.*